# FASCICULE DE BREVET EUROPEEN

(11) **EP 1 722 813 B1**
(45) Date de publication et mention de la délivrance du brevet: **09.02.2011**
(21) Numéro de dépôt: 05733011.0
(22) Date de dépôt: 02.03.2005
(51) Int. Cl.: A61K 38/48, A61P 17/00

(54) **UTILISATION COSMÉTIQUE OU THÉRAPEUTIQUE DE TOXINE BOTULIQUE POUR PREVENIR LA POUSSE DE POILS**
KOSMETISCHE ODER THERAPEUTISCHE VERWENDUNG VON BOTULINUMTOXIN ZUR VERHINDERUNG VON HAARWUCHS
COSMETIC OR THERAPEUTIC USE OF BOTULINUM TOXIN FOR PREVENTING HAIR GROWTH

(30) Priorité: 04.03.2004 EP 04290582
(43) Date de publication de la demande: 22.11.2006
(73) Titulaire: IPSEN PHARMA, 92100 Boulogne-Billancourt (FR); Le Louarn, Claude, 75116 Paris (FR)
(72) Inventeur: LE LOUARN, Claude, F-75116 Paris (FR)
(74) Mandataire: Retzler, Charlotte
(86) Numéro de dépôt international: PCT/FR2005/000495
(87) Numéro de publication internationale: WO 2005/087258

(56) Documents cités:
- FR-A- 2 813 532
- ASADA-KUBOTA M: "INHIBITION OF HAIR GROWTH BY SUBCUTANEOUS INJECTION OF A SYMPATHETIC NEUROTOXIN, 6-HYDROXYDOPAMINE IN NEONATAL MICE" ANATOMY AND EMBRYOLOGY, SPRINGER INTERNATIONAL, BERLIN, DE, vol. 191, no. 5, mai 1995 (1995-05), pages 407-414, XP000861802 ISSN: 0340-2061
- LE LOUARN C: "[The plastic surgeon and the prevention of facial aging process]" ANNALES DE CHIRURGIE PLASTIQUE ET ESTHETIQUE. OCT 2003, vol. 48, no. 5, octobre 2003 (2003-10), pages 346-349, XP002287709 ISSN: 0294-1260
- AZZIZ RICARDO: "The evaluation and management of hirsutism." OBSTETRICS AND GYNECOLOGY, vol. 101, no. 5 Part 1, mai 2003 (2003-05), pages 995-1007, XP002287710 ISSN: 0029-7844
- WENDELIN DANIEL S ET AL: "Hypertrichosis." JOURNAL OF THE AMERICAN ACADEMY OF DERMATOLOGY. FEB 2003, vol. 48, no. 2, février 2003 (2003-02), pages 161-179 ; qui, XP002287711 ISSN: 0190-9622

## Description

La présente invention a pour objet l'utilisation de toxine botulique pour préparer un médicament destiné à prévenir la pousse de poils. Elle concerne également une méthode cosmétique de prévention de la pousse de poils.

Chez les personnes ayant de l'hypertrichose ou les femmes atteintes d'hirsutisme, la pousse de poils représente une gêne importante. Par ailleurs, pour certaines personnes, notamment chez les femmes, la présence de poils sur certaines parties du corps est de toute façon perçue comme particulièrement inesthétique.

Il existe donc un besoin pour une méthode permettant de limiter de façon efficace et sans effets indésirables la repousse de poils chez les personnes mentionnées précédemment.

L'objet de la présente invention est d'offrir une solution particulièrement efficace tout en étant dénuée d'effets secondaires.

La toxine botulique, en particulier la toxine botulique de type A (Dysport^{®} commercialisé par Ipsen ou Botox^{®} commercialisé par Allergan), est utilisée depuis les années 80 chez l'homme pour le traitement de maladies / désordres divers et variés. Parmi les maladies / désordres pouvant être traités par la toxine botulique, on peut citer entre autres des désordres neuromusculaires (par exemple le blépharospasme, la spasticité de l'adulte ou de l'enfant ou encore le torticolis), la migraine, la douleur en général, des désordres urologiques (par exemple l'hypertrophie bénigne de la prostate, la rétention urinaire ou l'incontinence), le diabète, l'hyperhidrose (ou transpiration excessive), l'hypersalivation ou même les rides.

Selon l'invention, de la toxine botulique est utilisée pour prévenir la pousse de poils chez une personne ayant de l'hypertrichose ou chez une patiente atteinte d'hirsutisme, ou alors chez des personnes saines ou ayant éventuellement de l'hypertrichose qui souhaitent un traitement cosmétique pour prévenir la pousse de poils.

L'invention concerne donc en particulier l'utilisation de toxine botulique pour préparer un médicament destiné à prévenir la pousse de poils chez une personne ayant de l'hypertrichose ou chez une patiente atteinte d'hirsutisme.

L'invention concerne aussi une méthode de traitement cosmétique destinée à prévenir la pousse de poils chez une personne souhaitant un tel traitement, ladite méthode comprenant l'administration, au niveau de la zone à traiter, d'une composition pharmaceutique contenant de la toxine botulique en quantité efficace.

L'un des principaux avantages de l'invention réside dans l'effet relativement durable obtenu grâce à l'emploi de la toxine botulique. Ainsi, cet effet persiste d'une façon générale pendant au moins 3 ou 4 mois après l'administration de la toxine botulique (voire plus chez certains patients).

La toxine botulique utilisée pour la préparation d'un médicament selon l'invention sera choisie parmi les toxines botuliques de type A (incluant notamment A1 et A2), B, C (incluant notamment C1 et C2), D, E, F et G. De préférence, elle sera choisie parmi les toxines botuliques de type A, B et F. Encore plus préférentiellement, elle sera choisie parmi les toxines botuliques de type A et B ; en particulier, il s'agira de la toxine botulique de type A (et plus particulièrement de toxine botulique de type A1).

Par ailleurs, la toxine botulique utilisée pour la préparation d'un médicament selon l'invention pourra se présenter sous forme d'un complexe comprenant la toxine botulique et d'autres protéines ou alors sous forme libre (i.e. libre de toute protéine la complexant).

Selon l'invention, le médicament préparé pourra notamment être une poudre lyophilisée comprenant la toxine botulique (auquel cas le médecin reconstituera la solution avec de l'eau ou une solution aqueuse saline avant de l'injecter au patient) ou encore une solution injectable comprenant ladite toxine.

Les considérations qui précèdent concernant la toxine botulique utilisée pour préparer un médicament selon l'invention et le médicament obtenu sont applicables *mutatis mutandis* à la toxine botulique utilisée pour la méthode de traitement cosmétique selon l'invention et à l'agent cosmétique utilisé pour mettre en oeuvre ladite méthode de traitement cosmétique.

Le médicament préparé selon l'invention est destiné à être administré, par exemple par injection, au niveau des zones du patient affectées par la pilosité excessive qui auront été choisies par le médecin traitant, de préférence après que ces zones aient été rasées. De préférence, ces zones seront choisies parmi le groupe constitué par le visage, le thorax, l'abdomen, la région fessière, la région génito-crurale, les jambes et les bras. Pour le traitement cosmétique, l'on pourra par exemple injecter une composition liquide ou semi-liquide ou encore un gel comprenant la toxine botulique au niveau des zones où l'épilation aura été ou sera ensuite réalisée. Alternativement, un patch contenant de la toxine botulique pourra être appliqué, ou bien une crème contenant de la toxine botulique répartie, au niveau de la zone à traiter.

De préférence, la zone du corps à laquelle la méthode de traitement cosmétique sera appliquée sera choisie parmi le groupe constitué par le torse (par exemple les seins), les jambes (par exemple les cuisses), les bras, les aisselles et le visage (par exemple les arcades sourcilières, la zone située entre les lèvres et le nez [c'est-à-dire la lèvre supérieure blanche], le menton ou les joues).

En ce qui concerne la voie d'administration du médicament ou du traitement cosmétique selon l'invention, l'homme du métier pourra envisager n'importe quelle voie d'administration adaptée ; toutefois, ladite voie d'administration sera de préférence choisie de façon à ce que la toxine botulique, une fois administrée, soit essentiellement répartie au niveau de l'épiderme des zones à traiter.

Bien entendu, les utilisations selon l'invention pourront également être employées pour effectuer des traitements thérapeutiques ou cosmétiques destinés à des animaux domestiques dont la pilosité serait excessive ou gênerait leur maître.

La dose de toxine botulique à prévoir selon la présente invention variera suivant l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

A titre indicatif, pour la toxine botulique de type A1, la dose d'administration envisagée pour un médicament selon l'invention pourra être de 0,05 à 1 unités DL₅₀ de toxine botulique de type A1 par cm² de peau à traiter, de préférence de 0,1 à 0,5 unités DL₅₀ de toxine botulique de type A1 par cm² de peau à traiter et plus préférentiellement de 0,2 à 0,4 unités DL₅₀ de toxine botulique de type A1 par cm² de peau à traiter (par exemple environ 0,3 unités DL₅₀ de toxine botulique de type A1 par cm² de peau à traiter). Pour les toxines botuliques d'autres types, l'homme du métier adaptera la dose nécessaire dans la mesure où il connaît l'activité thérapeutique relative de chacune de ces toxines botuliques par rapport à la toxine botulique de type A1. Les unités DL₅₀ sont couramment utilisées par le praticien utilisant la toxine botulique ; une unité DL₅₀ de toxine botulique correspond à la dose de toxine équivalente tuant 50% d'un groupe de 18 à 20 souris femelles Swiss-Webster pesant environ 20 grammes chacune.

Le terme "environ" fait référence à un intervalle autour de la valeur considérée. Tel qu'utilisé dans la présente demande, "environ X" signifie un intervalle de X moins 10% de X à X plus 10% de X, et de préférence un intervalle de X moins 5% de X à X plus 5% de X.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

L'exemple suivant est présenté pour illustrer les procédures ci-dessus et ne doit en aucun cas être considéré comme une limite à la portée de l'invention.

### EXEMPLE

Une patiente de 39 ans souhaite diminuer le duvet au niveau de sa lèvre supérieure. Une solution contenant 3 unités de Botox^{®} (produit commercialisé par Allergan France ; la solution est préparée selon les instructions du fabricant) est injectée dans l'épiderme de cette patiente, en plusieurs points situés juste au dessus du vermillon de sa lèvre supérieure, les 3 unités étant équitablement réparties sur la zone traitée. Au contrôle, 4 mois après, la patiente présente un duvet de la lèvre supérieure nettement plus léger.

## Revendications

1. Utilisation de toxine botulique pour préparer un médicament destiné à prévenir la pousse de poils chez une personne ayant de l'hypertrichose ou chez une patiente atteinte d'hirsutisme.

2. Utilisation selon la revendication 1, **caractérisée en ce que** le médicament préparé est destiné à prévenir la pousse de poils chez une personne ayant de l'hypertrichose.

3. Utilisation selon la revendication 1, **caractérisée en ce que** le médicament préparé est destiné à prévenir la pousse de poils chez une patiente atteinte d'hirsutisme.

4. Utilisation selon l'une des revendications 1 à 3, **caractérisée en ce que** la toxine botulique utilisée est choisie parmi les toxines botuliques de type A, B et F.

5. Utilisation selon la revendication 4, **caractérisée en ce que** la toxine botulique utilisée est une toxine botulique de type A.

6. Méthode de traitement cosmétique destinée à prévenir la pousse de poils chez une personne souhaitant un tel traitement, ladite méthode comprenant l'administration, au niveau de la zone à traiter, d'une composition pharmaceutique contenant de la toxine botulique en quantité efficace.

7. Méthode de traitement cosmétique selon la revendication 6, **caractérisée en ce que** la toxine botulique utilisée est choisie parmi les toxines botuliques de type A, B et F.

8. Méthode de traitement cosmétique selon la revendication 7, **caractérisée en ce que** la toxine botulique utilisée est une toxine botulique de type A.

9. Méthode de traitement cosmétique selon l'une des revendications 6 à 8, **caractérisée en ce qu'**elle est appliquée à une zone du corps choisie parmi le groupe constitué par le torse, les jambes, les bras, les aisselles et le visage.

10. Méthode de traitement cosmétique destinée à prévenir la pousse de poils chez un animal domestique, ladite méthode comprenant l'administration, au niveau de la zone à traiter, d'une composition pharmaceutique contenant de la toxine botulique en quantité efficace.

## Claims

1. Use of botulinum toxin for the preparation of a medicament intended to prevent hair growth in a person with hypertrichosis or in a patient suffering from hirsutism.

2. Use according to claim 1, **characterized in that** the prepared medicament is intended to prevent hair growth in a person with hypertrichosis.

3. Use according to claim 1, **characterized in that** the prepared medicament is intended to prevent hair growth in a patient suffering from hirsutism.

4. Use according to one of claims 1 to 3, **characterized in that** the botulinum toxin used is chosen from the botulinum toxins of type A, B and F.

5. Use according to claim 4, **characterized in that** the botulinum toxin used is a botulinum toxin of type A.

6. Cosmetic treatment method intended to prevent hair growth in a person wanting such a treatment, said method comprising the administration, to the area to be treated, of a pharmaceutical composition containing botulinum toxin in an effective quantity.

7. Cosmetic treatment method according to claim 6, **characterized in that** the botulinum toxin used is chosen from the botulinum toxins of type A, B and F.

8. Cosmetic treatment method according to claim 7, **characterized in that** the botulinum toxin used is a botulinum toxin of type A.

9. Cosmetic treatment method according to one of claims 6 to 8, **characterized in that** it is applied to an area of the body chosen from the group constituted by the torso, the legs, the arms, the armpits and the face.

10. Cosmetic treatment method intended to prevent hair growth in a pet, said method comprising the administration, to the area to be treated, of a pharmaceutical composition containing botulinum toxin in an effective quantity.

## Patentansprüche

1. Verwendung von Botulinumtoxin zur Herstellung eines Medikaments, welches zur Prävention von Haarwuchs vorgesehen ist bei einer Person, die Hypertrichose hat, oder bei einer Patientin, die an Hirsutismus erkrankt ist.

2. Verwendung nach Anspruch 1, **dadurch gekennzeichnet, dass** das hergestellte Medikament zur Prävention von Haarwuchs bei einer Person vorgesehen ist, die Hypertrichose hat.

3. Verwendung nach Anspruch 1, **dadurch gekennzeichnet, dass** das hergestellte Medikament zur Prävention von Haarwuchs bei einer Patientin vorgesehen ist, die an Hirsutismus erkrankt ist.

4. Verwendung nach einem der Ansprüche 1 bis 3, **dadurch gekennzeichnet, dass** das verwendete Botulinumtoxin ausgewählt ist unter Botulinumtoxinen des Typs A, B und F.

5. Verwendung nach Anspruch 4, **dadurch gekennzeichnet, dass** das verwendete Botulinumtoxin Botulinumtoxin des Typs A ist.

6. Verfahren zur kosmetischen Behandlung zur Prävention von Haarwuchs bei einer Person, welche eine solche Behandlung wünscht, wobei das Verfahren die Verabreichung einer pharmazeutischen Zusammensetzung, welche eine wirksame Menge an Botulinumtoxin enthält, auf dem Gebiet der zu behandelnden Zone umfasst.

7. Verfahren zur kosmetischen Behandlung nach Anspruch 6, **dadurch gekennzeichnet, dass** das verwendete Botulinumtoxin ausgewählt ist unter Botulinumtoxinen des Typs A, B und F.

8. Verfahren zur kosmetischen Behandlung nach Anspruch 7, **dadurch gekennzeichnet, dass** dass das verwendete Botulinumtoxin Botulinumtoxin des Typs A ist.

9. Verfahren zur kosmetischen Behandlung nach einem der Ansprüche 6 ä 8, **dadurch gekennzeichnet, dass** es auf einer Körperzone angewendet wird, welche ausgewählt ist unter Oberkörper, den Beinen, den Armen, den Achselhöhlen und dem Gesicht.

10. Verfahren zur kosmetischen Behandlung zur Prävention von Haarwuchs bei Haustieren, wobei das Verfahren die Verabreichung einer pharmazeutischen Zusammensetzung, welche eine wirksame Menge and Botulinumtoxin enthält, auf dem Gebiet der zu behandelnden Zone umfasst.
